# EUROPÄISCHE PATENTANMELDUNG

(11) **EP 0 803 267 A2**
(43) Veröffentlichungstag der Anmeldung: **29.10.1997**
(21) Anmeldenummer: 97105592.6
(22) Anmeldetag: 04.04.1997
(51) Int. Cl.: A61M 39/22, A61M 39/14

(54) **Medizintechnische Anschlussvorrichtung**

(30) Priorität: 24.04.1996 DE 29607437 U
(71) Anmelder: B. BRAUN MELSUNGEN AG, 34212 Melsungen (DE)
(72) Erfinder: Fuchs, Jürgen, 34308 Bad Emstal (DE)
(74) Vertreter: Selting, Günther, Dipl.-Ing.

(57) **Zusammenfassung**

Die Anschlußvorrichtung weist einen Konnektor (10) mit Innenkegel (11) zum Einstecken des Außenkegels (31) eines Gegenkonnektors (30) auf. Ferner ist der Konnektor mit einem Brechsiegel (18) versehen, das den Kanal (13) versperrt. Beim Anschrauben des Gegenkonnektors (30) an den Konnektor (10) reißt das Brechsiegel (18) vom Konnektor (10) ab, so daß ein Flüssigkeitsdurchgang durch den Konnektor ermöglicht wird. Das Brechsiegel (18) ist mit einem Stab (20) versehen, der im Innenkegel (11) in axialem Abstand von dem Einsteckende endet und somit nur durch den Außenkegel (31) des Gegenkonnektors (10) zugänglich ist.

## Beschreibung

Die Erfindung betrifft eine medizintechnische Anschlußvorrichtung, insbesondere für Infusionsgeräte zur Überleitung einer Infusionsflüssigkeit von einem Flüssigkeitsbehälter zum Infusionsgerät.

Bekannt sind Anschlußvorrichtungen in Form von Luer-Lock-Konnektoren mit Innenkegel. Solche Konnektoren werden mit Gegenkonnektoren verbunden, die einen Luer-Lock-Außenkegel aufweisen. Die Konnektoren haben ein Brechsiegel, das den Flüssigkeitsdurchgang versperrt und von Hand abgebrochen werden kann, um den Flüssigkeitsdurchgang freizugeben. Das Brechsiegel bildet eine Originalitätsgarantie für die mit der Anschlußvorrichtung versehene Vorrichtung und deren Inhalt und ferner auch eine Kontaminationssperre. Bei einem Konnektor, bei dem das Brechsiegel von Hand abgebrochen wird, besteht die Gefahr des versehentlichen Abbrechens des Brechsiegels vor der Konnektion. Dabei kann unbeabsichtigt Flüssigkeit durch den Konnektor hindurch auslaufen.

Der Erfindung liegt die Aufgabe zugrunde, eine medizintechnische Anschlußvorrichtung mit Brechsiegel zu schaffen, bei der sichergestellt ist, daß das Brechsiegel nicht unbeabsichtigt zerreißt.

Die Lösung dieser Aufgabe erfolgt erfindungsgemäß mit den im Patentanspruch 1 angegebenen Merkmalen.

Bei der erfindungsgemäßen Anschlußvorrichtung ist das Brechsiegel in oder an dem Konnektor an einer Stelle enthalten, an der es nicht von außen zugänglich ist. Das Brechsiegel weist einen mit dem Konnektor über eine Sollbruchlinie verbundenen Sperrkörper auf, von dem ein Stab in Richtung auf das Einsteckende des Innenkegels absteht. Dieser Stab endet im Konnektor in axialem Abstand von dem Einsteckende, so daß er mit den Fingern nicht erreichbar ist, beim Einstecken des Außenkegels eines Gegenkonnektors jedoch bewegt wird und abreißt. Damit wird erreicht, daß beim Einstecken des Außenkegels des Gegenkonnektors zugleich automatisch das Brechsiegel abreißt. Da der Stab sich im Innenkegel des Konnektors befindet, ist er mit der Hand nicht zugänglich, so daß er nur mit dem Gegenkonnektor erreicht und betätigt werden kann.

Die erfindungsgemäße Anschlußvorrichtung eignet sich insbesondere für Infusionsbehältnisse, z.B. Beutel, die eine Infusionsflüssigkeit enthalten. Durch das Brechsiegel wird der Beutelinhalt originalitätsgetreu verschlossen. Andererseits ist die Anschlußvorrichtung aber auch für Schläuche und andere Flüssigkeits-Überleitungsvorrichtungen geeignet.

Im folgenden wird unter Bezugnahme auf die Zeichnungen ein Ausführungsbeispiel der Erfindung näher erläutert.

Es zeigen:
- Fig. 1: einen Längsschnitt durch den Konnektor im Originalitätszustand und
- Fig. 2: einen Längsschnitt durch den Konnektor mit eingestecktem Gegenkonnektor bei abgerissenem Sperrkörper.

Die dargestellte Anschlußvorrichtung weist einen Konnektor 10 in Form eines länglichen Rohres auf, das mit einem Innenkegel 11 versehen ist. Das eine Ende des Innenkegels 11 bildet das Einsteckende 12 für einen Gegenkonnektor. An den Innenkegel 11 schließt sich ein zylindrischer Rohrabschnitt 13 an, der im Innern eines Ansatzes 14 verläuft. Auf den Ansatz 14 ist ein Schlauch 15 aufgesteckt und mit dem Ansatz 14 verklebt. Der Konnektor 10 weist einen Ringkragen 16 auf, gegen den das Ende des Schlauchs 15 stößt. Der Schlauch 15 ist hier Bestandteil eines Infusionsbeutels, der eine Infusionslösung enthält. Der Konnektor 10 dient dazu, den Infusionsbeutel an eine Schlauchleitung anzuschließen, die zu einem Infusionsgerät führt.

Der Konnektor 10 ist als Luer-Lock-Konnektor ausgebildet. Er ist in der Nähe seines Einsteckendes 12 außen mit Gewindeteilen 17 versehen, auf die ein Gegenkonnektor aufgeschraubt werden kann.

Der Konnektor 10 ist mit einem Brechsiegel 18 versehen, das aus einem Sperrkörper 19 und einem sich daran anschließenden Schaft 20 besteht. Der Sperrkörper 19 ist an dem dem Einsteckende 12 abgewandten Ende 21 des Konnektors in Verlängerung des Kanals 13 angeordnet. Der Sperrkörper 19 steht über das Ende 21 hinaus vor, während der Schaft 20 sich durch den Kanal 13 bis in den Innenkegel 11 hinein erstreckt.

Der Sperrkörper 19 hat generell die Gestalt eines Kegels, wobei der Stab 20 sich an die Basisfläche des Kegels anschließt. Der Umfang der Basisfläche des Kegels ist über eine aus einer Filmhaut bestehenden Sollbruchlinie 22 mit dem Ansatz 14 verbunden. Die Sollbruchlinie 22 bildet einen geschlossenen Ring, der zusammen mit dem Sperrkörper 19 den Kanal 13 flüssigkeitsdicht verschließt und die einzige Verbindung des Brechsiegels 18 mit dem Konnektorkörper darstellt. Die Kegelfläche des Sperrkörpers 19 ist mit einer Rippenstruktur 11 versehen, welche verhindert, daß das von dem Konnektor 10 abgerissene Brechsiegel 18, falls dieses im Infusionsbeutel herumschwimmt, sich verschließend gegen das Ende 21 des Konnektors legen kann. Der Stab 20 erstreckt sich koaxial in dem Konnektor, wobei zwischen dem Umfang des Stabes und dem Kanal 13 ein Freiraum vorhanden ist, so daß der Stab den Kanal 13 nicht verschließt. Der Stab 20 ist mit einem Rippenprofil 23 versehen, um sicherzustellen, daß der Stab den Flüssigkeitsdurchgang nicht versperrt.

Der Stab 20 endet im Abstand von dem Einsteckende 12 im Innern des Innenkegels 11. Das Einsteckende 12 ist mit einem kontaminationsdichten abreißbaren Siegel 24 verschlossen, um die Sterilität des Innenraumes des Konnektors 10 zu gewährleisten.

Der Konnektor 10 ist zusammen mit dem Brechsiegel 18 einstückig aus Kunststoff hergestellt, wobei die Sollbruchlinie 22 integraler Bestandteil des Kunststoffteiles ist.

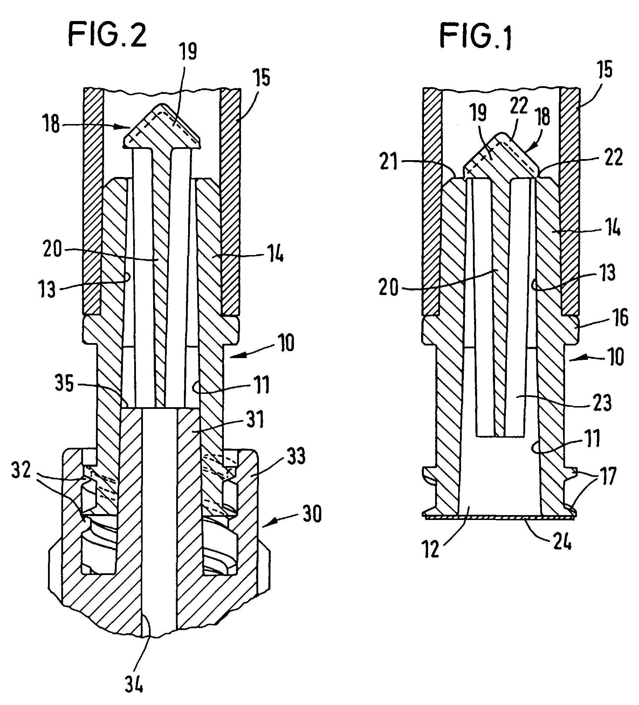

In Fig. 2 ist der Zustand dargestellt, daß ein Gegenkonnektor 30 an den Konnektor 10 angeschlossen ist. Der Gegenkonnektor 30 weist einen Außenkegel 31 auf, der in den Innenkegel 10 passend eingeschoben ist, und eine mit Innengewinde 32 versehene Schraubmuffe 33, die auf die Gewindeteile 17 des Konnektors 10 aufgeschraubt ist. Durch den Gegenkonnektor 30 erstreckt sich in Längsrichtung ein Kanal 34 bis zum vorderen Ende 35 des Außenkegels 31.

Beim Verbinden des Gegenkonnektors 30 mit dem Konnektor 10 wird der Außenkegel 31 in den Innenkegel 11 eingeschoben, wobei er gegen das Ende des Stabes 20 stößt. Nachdem die Gewindeteile 17 mit dem Innengewinde 32 in Eingriff gelangt sind, wird der Gegenkonnektor 30 gedreht, während der Konnektor 10 festgehalten wird. Dabei dringt der Außenkegel 31 im Innenkegel 11 weiter vor, wobei das Brechsiegel 18 an der Sollbruchlinie 22 abreißt. Hierbei wird der Sperrkörper 19 von dem Ansatz 14 weggedrückt und abgelöst. Durch das Rippenprofil des Stabes 20 ist sichergestellt, daß in dieser Position der Kanal 34 von dem Stab 20 nicht versperrt wird.

Sollte das Brechsiegel 18 nach dem Öffnen in das Infusionsbehältnis gelangen und bei der Infusion durch entstehende Sogwirkung um 180° verdreht wieder auf dem Konnektor 10 aufsetzen, so ermöglicht die Rippenstruktur 22 den Durchfluß der Infusionsflüssigkeit.

## Patentansprüche

1. Medizintechnische Anschlußvorrichtung mit einem Konnektor (10), der einen Innenkegel (11) mit einem Einsteckende (12) zum Einführen des Außenkegels (31) eines Gegenkonnektors (30) aufweist, und mit einem den Durchgang durch den Konnektor (10) versperrenden Brechsiegel (18),
**dadurch gekennzeichnet,**
daß das Brechsiegel (18) aus einem Sperrkörper (19) besteht, der mit dem Konnektor (10) über eine Sollbruchlinie (22) verbunden ist und der einen in Richtung auf das Einsteckende (12) abstehenden Stab (20) aufweist, welcher im Innenkegel (11) in axialem Abstand von dem Einsteckende (12) endet.

2. Anschlußvorrichtung nach Anspruch 1, dadurch gekennzeichnet, daß der Sperrkörper (19) an dem dem Einsteckende (12) abgewandten Ende (21) des Konnektors (10) angeordnet ist.

3. Anschlußvorrichtung nach Anspruch 1 oder 2, dadurch gekennzeichnet, daß der Sperrkörper (19) eine Rippenstruktur (22) aufweist.

4. Anschlußvorrichtung nach einem der Ansprüche 1-3, dadurch gekennzeichnet, daß der Stab (20) mindestens an seinem dem Einsteckende (12) zugewandten Ende ein Rippenprofil (23) hat.

5. Anschlußvorrichtung nach einem der Ansprüche 1-4, dadurch gekennzeichnet, daß der Sperrkörper (19) und der Stab (20) einstückig dem Konnektor (10) ausgebildet sind und die Sollbruchlinie (22) aus einer Filmhaut besteht.

6. Anschlußvorrichtung nach einem der Ansprüche 1-5, dadurch gekennzeichnet, daß der Stab (20) sich von dem Sperrkörper (19) aus zum Einsteckende (12) verjüngt.
